(19) Europäisches Patentamt
European Patent Office
Office européen des brevets

(11) Publication number: **0 028 803**
**A1**

# (12) EUROPEAN PATENT APPLICATION

(21) Application number: 80106792.7

(22) Date of filing: 05.11.80

(51) Int. Cl.$^3$: C 07 D 501/22
C 07 D 499/68, C 07 H 13/12
A 61 K 31/70

(30) Priority: 09.11.79 IT 2714779

(43) Date of publication of application:
20.05.81 Bulletin 81/20

(84) Designated Contracting States:
AT BE CH DE FR GB IT LI LU NL SE

(71) Applicant: **Simes S.p.A.**
**Via Bellerio 41**
**I-20161 Milano(IT)**

(72) Inventor: **Ferrari, Giorgio**
**via Borgonuovo, 7**
**I-20121 Milano(IT)**

(72) Inventor: **Vecchietti, Vittorio**
**via Spartaco, 23**
**I-20135 Milano(IT)**

(74) Representative: **Marchi, Massimo et al,**
**c/o Zambon S.p.A. Patent & Licensing Dept. Via Lillo del Duca, 10**
**I-20091 Bresso (Milano)(IT)**

## (54) New penicillanic and cephalosporanic derivatives, preparation thereof and pharmaceutical compositions containing same.

(57) The invention relates to a new penicillanic and cephalosporanic derivatives of the general formula (I)

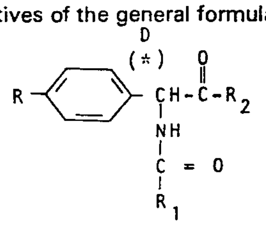

wherein R is H or -OH; $R_1$ is a carbohydrate selected from the class of hexoses comprising glucose, galactose, glucose acetals, glucose chetals, galactose acetals and galactose chetals, and $R_2$ is

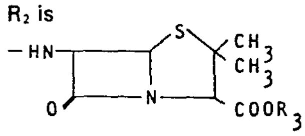

or

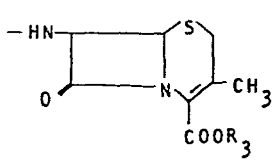

$R_3$ being H or an alkaline or alkaline-earth metal, as well as to the preparation thereof and pharmaceutical compositions containing same.

A process according to the invention comprises the reaction of a compound of the formula

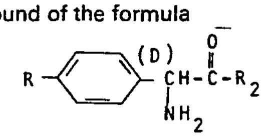

with a carbohydrate, selected from the class of hexoses, in cyclic form, in an aqueous medium, in the presence of a water-miscible organic solvent, in the presence of a base, the reaction product being optionally transformed into the derivative wherein $R_1$ is a carbohydrate in the linear form by treatment with Lewis' acid or a hydrogen halide acid.

The compounds according to the invention are used in pharmaceutical compositions useful for treatment of infections by gram-positive and gram-negative pathogenous bacteria.

EP 0 028 803 A1

Croydon Printing Company Ltd.

The present invention relates to new penicillanic and cephalosporanic derivatives, more particularly to optically active forms (D-isomers) of these compounds and to the preparation and use thereof.

The compounds according to the invention have the general formula (I)

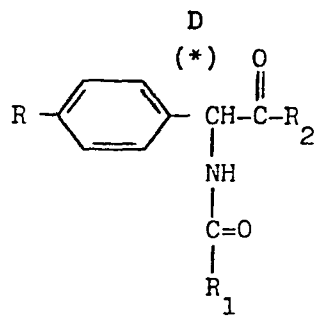

(I)

wherein R is H or -OH; $R_1$ is a radical of a carbohydrate selected from the class of hexoses comprising glucose, galactose, glucose acetals, glucose chetals, galactose acetals and galactose chetals, $R_2$ is a group selected from the group comprising

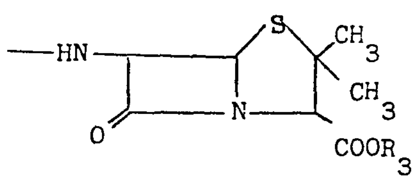

and

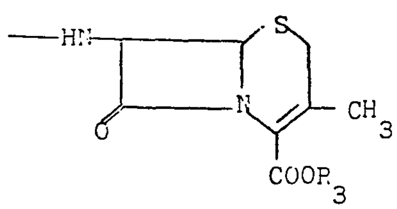

wherein $R_3$ is H or an alkaline or earth-alkaline metal. The derivative of the hexose are prepared by reacting it with a suitable reagent like benzaldehyde, acetone and cyclohexanone.

Both the cyclic and the linear form of the hexoses fall under the

scope of this invention.

When the hexose is in cyclic form the radical $R_1$ may be represented by the following formulae

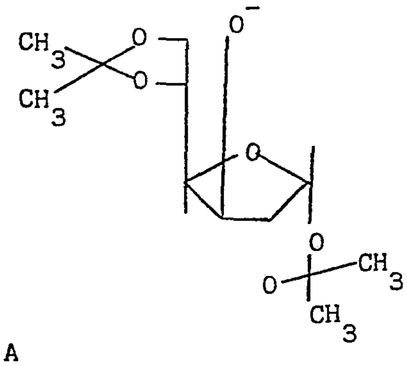

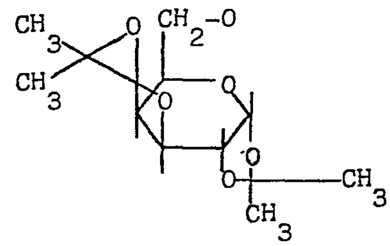

A B

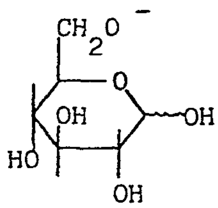

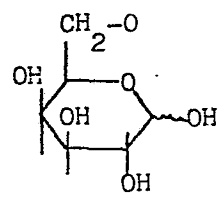

C D

The following compounds fall under the scope of the present invention:

(1) 6β-[1,2-3,4-diisopropylidene-D-galactopyranoside-6-O-carbonyl-amino-D-phenylacetyl]-amino-3,3-dimethyl-7-oxo-4-thia-1-azabicyclo-[3,2,0]-heptan-2-carboxylic acid.

(2) 7β-[1,2-3,4-diisopropylidene-D-galactopyranoside-6-O-carbonyl-amino-D-phenylacetyl]-amino-3-methyl-8-oxo-5-thia-1-azabicyclo-[4,2,0]-oct-2-en-2-carboxylic acid.

(3) 6β-[1,2-5,6-diisopropylidene-D-glucofuranoside-3-O-carbonyl-amino-D-phenylacetyl]-amino-3,3-dimethyl-7-oxo-4-thia-1-azabicyclo-[3,2,0]-heptan-2-carboxylic acid.

(4) 7β-[1,2-5,6-diisopropylidene-D-glucofuranoside-3-O-carbonyl--amino-D-phenylacetyl]-amino-3-methyl-8-oxo-5-thia-1-azabicyclo-[4,2,0]-oct-2-en-2-carboxylic acid.

(5) 7β-(D-galactosyl-3-O-carbonylamino-D-phenylacetyl)-amino-3-

-methyl-8-oxo-5-thia-1-azabicyclo-/4,2,0/-oct-2-en-2-carboxylic acid.

(6) 6β-(D-galactosyl-3-O-carbonylamino-D-phenylacetyl)-amino-3,3-dimethyl-7-oxo-4-thia-1-azabicyclo-/3,2,0/-heptan-2-carboxylic acid.

(7) 7β-(D-glucosyl-6-O-carbonylamino-D-phenylacetyl)-amino-3-methyl-2-oxo-5-thia-1-azabicyclo-/4,2,0/-oct-2-en-2-carboxylic acid.

(8) 6β-(D-glucosyl-6-O-carbonylamino-D-phenylacetyl)-amino-3,3-dimethyl-7-oxo-4-thia-1-azabicyclo-/3,2,0/-heptan-2-carboxylic acid.

(9) 6β-/1,2-3,4-diisopropylidene-D-galactopyranoside-6-O-carbonylamino-D-p-hydroxyphenylacetyl/-amino-3,3-dimethyl-7-oxo-4-thia-1-azabicyclo-/3,2,0/-heptan-2-carboxylic acid.

(10) 6β-/1,2-5,6-diisopropylidene-D-glucofuranoside-3-O-carbonylamino-D-p-hydroxyphenylacetyl/-amino-3,3-dimethyl-7-oxo-4-thia-1-azabicyclo-/3,2,0/-heptan-2-carboxylic acid.

(11) 6β-(D-galactosyl-3-O-carbonylamino-D-p-hydroxyphenylacetyl)-amino-3,3-dimethyl-7-oxo-4-thia-1-azabicyclo-/3,2,0/-heptan-carboxylic acid.

(12) 6β-(D-glucosyl-6-O-carbonylamino-D-p-hydroxyphenylacetyl)-amino-3,3-dimethyl-7-oxo-4-thia-1-azabicyclo-/3,2,0/-heptan-2-carboxylic acid.

The Applicant has found that the compounds of the general formula (I), when $R_1$ is the radical A or B, can be prepared by reacting a compound of the general formula (II)

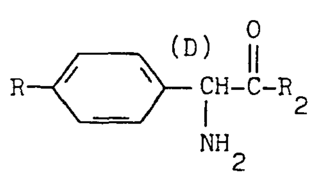

(II)

(wherein R and $R_2$ have the previously specified meanings and $R_3$ is H, an alkaline or earth-alkaline ion) with a compound of the formula (IIIA) or (IIIB) according to whether $R_1$ is A or B

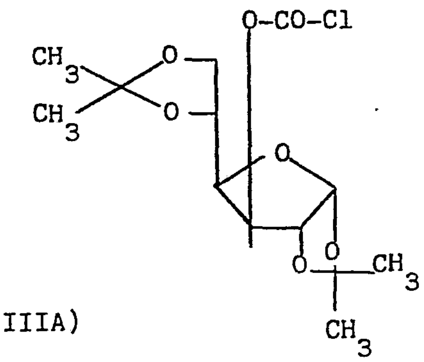

(IIIA)

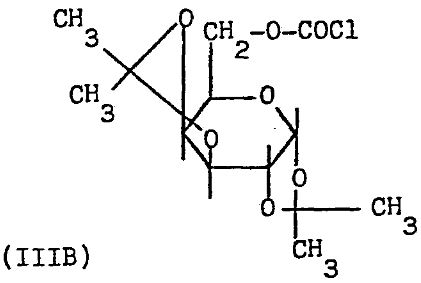

(IIIB)

The reaction is carried out in an aqueous medium in the presence of water-miscible organic solvents such as acetone, tetrahydrofurane, dioxane, diglyme, at temperatures comprised between -10 °C and +10 °C, in the presence of a molar equivalent of an inorganic base selected from the group comprising NaOH, $Na_2CO_3$, $K_2CO_3$, $NaHCO_3$, $KHCO_3$, or an organic base selected from the group comprising pyridine, quinoline and triethylamine.

Furthermore, the Applicant has found that the compounds of the general formula (I), wherein $R_1$ may be the radical C or the radical D, can be prepared from the compounds of the general formula (I) wherein $R_1$ is the radical A or the radical B respectively by treatment in an aprotic chlorinated solvent selected from $CH_2Cl_2$, $CHCl_3$, $CCl_4$, tetrachloroethane, trichloroethylene, with an excess of a Lewis' acid selected from $BCl_3$, $BBr_3$, $BF_3$, $AlCl_3$, $FeCl_3$, or with a hydrogen halide acid selected from HCl and HBr, dissolved in a 1-4 C alcoholic solvent, at temperatures comprised between -30 °C and +10 °C.

The Applicant has found that the compounds of the general formula (I) are endowed with a remarkable antibacteric activity against gram-positive and gram-negative pathogenous bacteria, such as for

instance Staphylococcus albus, Bacillus pumilus, Proteus morgani, Escherichia coli ATCC 8739.

As example, the M.I.C. (minimum inhibitory concentration) of compound 3, determined according to the method described by Marimount-Wentz in Am. J. Clin. Path. 45, 548, (1960) and by Mac Lowry et al. in Appl. Microbiol. 20, 46, (1970) against S. albus is 0.48 mcg/ml; B. pumilus 1.95 mcg/ml; P. morgani 15.6 mcg/ml; E. coli 15.6 mcg/ml.

Ampicilline shows a M.I.C. against the same bacteria equal to 3.9 mcg/ml; 1.95 mcg/ml, 15.6 mcg/ml and 31.2 mcg/ml respectively.

Therefore, it is possible to use the compounds of the general formula (I) or salt thereof acceptable from the physiological point of view for the treatment of bacterial infections. The dosages are comprised between 1 and 100 mg/kg/die by oral or parenteral route administered in a single dose or by dividing the daily dosage into two or four administrations.

The pharmaceutical compositions of the present invention contain the compounds of the general formula (I) and optionally an inert carrier acceptable from the physiological point of view.

The preparations can be made up, for example, as capsules, tablets, syrups suitable for oral administration, or sterile aqueous solutions, or as lyophilized powder for parenteral administration. Obviously, the formulations are prepared according to the usual pharmaceutical procedure.

The following examples illustrate the preparation of the compounds according to the present invention but they are in any way limitative and expecially as to the preparation methods.

Example 1

2.017 g ampicilline . $3H_2O$ are dissolved in 100 ml $H_2O$ by the aid

of 925 mg $NaHCO_3$.

The solution is cooled to +5 °C and added with 1.6 g 1,2-3,4-diisopropylidene-6-O-chloroformyl-D-galactopyranoside dissolved in 20 ml acetone.

The whole is stirred for 2 hrs. at +5 °C and thereafter is extracted with ether (3x50 ml) and finally acidified with diluted HCl, at 0 °C, up to pH 2.

A further extraction is then carried out with $CH_2Cl_2$ (3x50 ml) followed by washing with water, drying on $Na_2SO_4$ and evaporation under vacuum to dryness. The obtained amorphous solid is crystallized from ethylacetate/petroleum ether (1:1). 1.5 g of 6β-/1,2-3,4-diisopropylidene-D-galactopyranoside-6-O-carbonylamino-D-phenylacetyl/-amino-3,3-dimethyl-7-oxo-4-thia-1-azabicyclo-/3,2,0/-heptan-2-carboxylic acid are thus obtained (compound 1).

m.p. = 158-160 °C

Analysis for $C_{29}H_{37}N_3O_{11}S$:

| | C | H | N |
|---|---|---|---|
| calculated (%) | 54.79 | 5.87 | 6.61 |
| found (%) | 54.76 | 5.94 | 6.32 |

U.V. (95% EtOH + 1 eq. NaOH) 212 mμ (ε 55,500)

$[\alpha]_D^{20}$ = +57.6° (c = 1 in 95% EtOH + 1 eq. NaOH)

By working as above described, the hereinbelow specified compounds are prepared.

- compound 2:

  7β-/1,2-3,4-diisopropylidene-D-galactopyranoside-6-O-carbonylamino-D-phenylacetyl/-amino-3-methyl-8-oxo-5-thia-azabicyclo-/4,2,0/-oct-2-en-2-carboxylic acid;

  m.p. = 158-165 °C (ethylacetate)

  Analysis for $C_{29}H_{35}N_3O_{11}S$:

| | C | H | N |
|---|---|---|---|
| calculated (%) | 54.97 | 5.57 | 6.63 |
| found (%) | 54.02 | 5.46 | 6.20 |

U.V. (95% EtOH + 1 eq. NaOH) 258 mμ (ε 12,000) 212 mμ (ε 58,000)

$[\alpha]_D^{20}$ = +17.4° (c = 1 in 95% EtOH + 1 eq. NaOH)

- compound 3:

6β-[1,2-5,6-diisoproylidene-D-glucofuranoside-3-O-carbonylamino-D-phenylacetyl]-3,3-dimethyl-7-oxo-4-thia-1-azabicyclo-[3,2,0]-heptan-2-carboxylic acid; amorphous;

m.p. = 130-150 °C

Analysis for $C_{29}H_{37}N_3O_{11}S$:

| | C | H | N |
|---|---|---|---|
| calculated (%) | 54.79 | 5.87 | 6.61 |
| found (%) | 55.40 | 5.94 | 6.32 |

U.V. (95% EtOH + 1 eq. NaOH) 211 mμ (ε 55,000)

$[\alpha]_D^{20}$ = +49° (c = 1 in 95% EtOH + 1 eq. NaOH)

- compound 4:

7β-[1,2-5,6-diisopropylidene-D-glucofuranoside-3-O-carbonyl-amino-D-phenylacetyl]-amino-3-methyl-8-oxo-5-thia-1-azabicyclo-[4,2,0]-oct-2-en-2-carboxylic acid; amorphous;

m.p. = 150-160 °C

Analysis for $C_{29}H_{35}N_3O_{11}S$:

| | C | H | N |
|---|---|---|---|
| calculated (%) | 54.97 | 5.57 | 6.63 |
| found (%) | 55.11 | 5.57 | 6.48 |

U.V. (95% EtOH + 1 eq. NaOH) 257 mμ (ε-12,700) 212 mμ (ε 66,500);

$[\alpha]_D^{20}$ = +29.5° (c = 1 in 95% EtOH + 1 eq. NaOH)

Example 2

A solution of compound 2 in 60 ml chloroform is cooled to -5 °C and treated with 2.5 ml of anhydrous hydrochloric acid dissolved in ethyl alcohol (21.4% w/v). The mixture is left to stand for 48 hr at +4 °C and thereafter the liquid is decanted from the precipitated tacky product.

The product is washed twice by decantation with chloroform and then is solidified by rubbing in the presence of isopropyl ether. The formed solid is filtered off, washed with isopropyl ether and dried under vacuum.

1.9 g of 7β-(D-galactosyl-3-O-carbonylamino-D-phenylacetyl)-amino--3-methyl-8-oxo-5-thia-1-azabicyclo-/4,2,0/-oct-2-en-2-carboxylic acid are thus obtained; (compound 5).

m.p. = 140 °C (decomposition)

Analysis for $C_{23}H_{29}N_3O_{12}S$:

| | C | H | N |
|---|---|---|---|
| calculated (%) | 48.28 | 5.07 | 7.34 |
| found (%) | 48.36 | 5.15 | 7.11 |

U.V. (95% EtOH + 1 eq. NaOH) 304 mμ (ε 4,200)

By working according to the above described the hereinbelow compounds are prepared.

- compound 6:

  6β-(D-galactosyl-3-O-carbonylamino-D-phenylacetyl)-amino-3,3--dimethyl-7-oxo-4-thia-1-azabicyclo-/3,2,0/-heptan-2-carboxylic acid.

  m.p. = 150 °C (decomposition)

  Analysis for $C_{23}H_{31}N_3O_{12}S$:

| | C | H | N |
|---|---|---|---|
| calculated (%) | 48.11 | 5.40 | 7.32 |
| found (%) | 47.15 | 5.42 | 6.95 |

- compound 7:

7β-(D-glucosyl-6-O-carbonylamino-D-phenylacetyl)-amino-3-methyl-2-oxo-5-thia-1-azabicyclo-/4,2,0/-oct-2-en-2-carboxylic acid.

m.p. = 150 °C (decomposition)

Analysis for $C_{23}H_{29}N_3O_{12}S$:

| | C | H | N |
|---|---|---|---|
| calculated (%) | 48.28 | 5.42 | 7.34 |
| found (%) | 48.40 | 6.95 | 7.40 |

U.V. (95% EtOH + 1 eq. NaOH) 304 mμ (ε 3,600)

- compound 8:

6β-(D-glucosyl-6-O-carbonylamino-D-phenylacetyl)-amino-3,3-dimethyl-7-oxo-4-thia-1-azabicyclo-/3,2,0/-heptan-2-carboxylic acid;

m.p. = 135 °C (decomposition)

Analysis for $C_{23}H_{31}N_3O_{12}S$:

| | C | H | N |
|---|---|---|---|
| calculated (%) | 48.11 | 5.40 | 7.32 |
| found (%) | 47.57 | 5.78 | 7.15 |

Claims:

1. New penicillanic and cephalosporanic derivatives of the general formula (I)

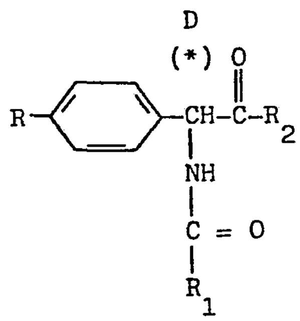

wherein R is H or -OH; $R_1$ is a carbohydrate radical, selected from the class of hexoses, comprising glucose, galactose, glucose acetals, glucose chetals, galactose acetals and galactose chetals,

$R_2$ is a group selected from

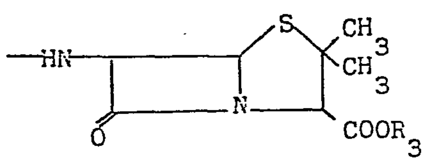

or

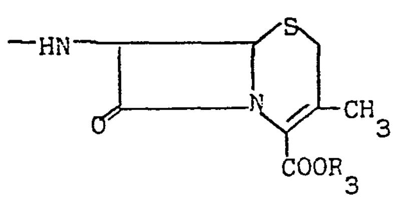

and $R_3$ is H or an alkaline or earth-alkaline metal.

2. New penicillanic and cephalosporanic derivatives according to claim 1, characterized in that the derivatives of the hexose are prepared by reacting it with a suitable reagent selected from the group consisting of benzaldehyde, acetone and cyclohexanone.

3. 6β-/1,2-3,4-diisopropylidene-D-galactopyranoside-6-O-carbonyl-

amino-D-phenylacetyl]-amino-3,3-dimethyl-7-oxo-4-thia-1-azabicyclo-[3,2,0]-heptan-2-carboxylic acid.

4. 7β-[1,2-3,4-diisopropylidene-D-galactopyranoside-6-O-carbonylamino-D-phenylacetyl]-amino-3-methyl-8-oxo-5-thia-1-azabicyclo-[4,2,0]-oct-2-en-2-carboxylic acid.

5. 6β-[1,2-5,6-diisopropylidene-D-glucofuranoside-3-O-carbonylamino-D-phenylacetyl]-amino-3,3-dimethyl-7-oxo-4-thia-1-azabicyclo-[3,2,0]-heptan-2-carboxylic acid.

6. 7β-[1,2-5,6-diisopropylidene-D-glucofuranoside-3-O-carbonyl-amino-D-phenylacetyl]-amino-3-methyl-8-oxo-5-thia-1-azabicyclo-[4,2,0]-oct-2-en-2-carboxylic acid.

7. 7β-(D-galactosyl-3-O-carbonylamino-D-phenylacetyl)-amino-3-methyl-8-oxo-5-thia-1-azabicyclo-[4,2,0]oct-2-en-2-carboxylic acid.

8. 6β-(D-galactosyl-3-O-carbonylamino-D-phenylacetyl)-amino-3,3-dimethyl-7-oxo-4-thia-1-azabicyclo-[3,2,0]-heptan-2-carboxylic acid.

9. 7β-(D-glucosyl-6-O-carbonylamino-D-phenylacetyl)-amino-3-methyl-2-oxo-5-thia-1-azabicyclo-[4,2,0]-oct-2-en-2-carboxylic acid.

10. 6β-(D-glucosyl-6-O-carbonylamino-D-phenylacetyl)-amino-3,3-dimethyl-7-oxo-4-thia-1-azabicyclo-[3,2,0]-heptan-2-carboxylic acid.

11. 6β-[1,2-3,4-diisopropylidene-D-galactopyranoside-6-O-carbonylamino-D-p-hydroxyphenylacetyl]-amino-3,3-dimethyl-7-oxo-4-thia-1--azabicyclo-1-[3,2,0]-heptan-2-carboxylic acid.

12. 6β-[1,2-5,6-diisopropylidene-D-glucofuranoside-3-O-carbonylamino-D-p-hydroxyphenylacetyl]-amino-3,3-dimethyl-7-oxo-4-thia-1--azabicyclo-[3,2,0]-heptan-2-carboxylic acid.

13. 6β-(D-galactosyl-3-O-carbonylamino-D-p-hydroxyphenylacetyl)-amino-3,3-dimethyl-7-oxo-4-thia-1-azabicyclo-[3,2,0]-heptan-2-carboxylic acid.

14. 6β-(D-glucosyl-6-O-carbonylamino-D-p-hydroxyphenylacetyl)-amino-3,3-dimethyl-7-oxo-4-thia-1-azabicyclo-/3,2,0/-heptan-2-carboxylic acid.

15. A process for the preparation of compounds of the general formula (I), characterized in that a compound of the general formula (II)

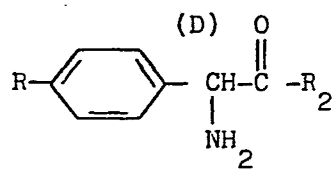

(wherein R and $R_2$ have the above specified meanings and $R_3$ is H, an alkaline or earth-alkaline ion) is reacted with a compound of the formula (IIIA) or (IIIB)

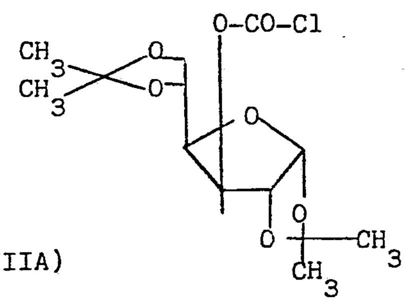

(IIIA)

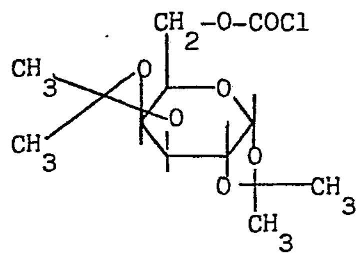

(IIIB)

in an aqueous medium in the presence of a water miscible organic solvent, at a temperature comprised between -10 °C and +10 °C, in the presence of a base.

16. A process according to claim 11, characterized in that the water miscible organic solvent is selected from the group consisting of acetone, tetrahydrofurane, dioxane, diglyme.

17. A process according to claim 11, characterized in that the base is an inorganic base selected from the group consisting of NaOH, $Na_2CO_3$, $K_2CO_3$, $NaHCO_3$ and $KHCO_3$.

18. A process according to claim 11, characterized in that the base is an organic base selected from the group consisting of pyridine, quinoline, triethylamine.

19. A process according to claims 11, 13 and 14, characterized in that the base is present in an amount corresponding to a molar equivalent.

20. A process according to claim 11, characterized in that the reaction product is treated in a chlorinated aprotic solvent with an excess of a reagent selected from Lewis' acids and hydrogen halide acids, the acid being dissolved in a 1-4 C alcoholic solvent, the reaction being carried out at a temperature comprised between -30 °C and +10 °C.

21. A process according to claim 16, characterized in that the chlorinated aprotic solvent is selected from the group consisting of $CH_2Cl_2$, $CHCl_3$, $CCl_4$, ethanetetrachloride, trichloroethylene.

22. A process according to claim 16, characterized in that the Lewis' acid is selected from the group consisting of $BCl_3$, $BBr_3$, $BF_3$, $AlCl_3$, $FeCl_3$.

23. A process according to claim 16, characterized in that the hydrogen halide acid is selected from HCl and HBr.

24. A process according to claim 16, characterized in that an excess of Lewis' acid or the hydrogen halide acid is used.

25. Pharmaceutical composition endowed with antibacterial activity, characterized in that it contains as active ingredient, a compound of the general formula (I) together with usually used excipients and vehicles.

26. Pharmaceutical composition according to claim 21, characterized in that the compound of the general formula (I) is contained in form of a pharmaceutically acceptable salt.

27. Pharmaceutical composition according to claim 21, characterized in that the compound of the formula (I) is in an amount comprised between 1 and 100 mg/kg.

28. Pharmaceutical composition according to claims from 21 to 23, characterized in that it is formulated as capsule, tablet or syrup for oral administration together with an inert pharmacologically acceptable carrier.

29. Pharmaceutical composition according to claim 21, characterized in that it is formulated as a sterile aqueous solution or lyophylized powder for parenteral administration.

0028803

European Patent Office

# EUROPEAN SEARCH REPORT

Application number
EP 80 10 6792

## DOCUMENTS CONSIDERED TO BE RELEVANT

| Category | Citation of document with indication, where appropriate, of relevant passages | Relevant to claim |
|---|---|---|
| | FR - A - 2 262 968 (OGURA)<br>* Pages 1,2; examples 6,7,9,10, 13,15-17,28 *<br>& GB - A - 1 463 282<br>& US - A - 4 025 622<br>---- | 1,25-29 |

CLASSIFICATION OF THE APPLICATION (Int. Cl.3)

C 07 D 501/22
499/68
C 07 H 13/12
A 61 K 31/70

TECHNICAL FIELDS SEARCHED (Int. Cl.3)

C 07 D 501/22
499/68
C 07 H 13/12

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 30-01-1981 | LUYTEN |

EPO Form 1503.1 06.78